# EUROPÄISCHE PATENTSCHRIFT

(11) **EP 1 925 260 B1**
(45) Veröffentlichungstag und Bekanntmachung des Hinweises auf die Patenterteilung: **12.01.2011**
(21) Anmeldenummer: 07022292.2
(22) Anmeldetag: 16.11.2007
(51) Int. Cl.: A61B 17/34

(54) **Vorrichtung zum klemmenden Festlegen an zylindrischen Bauteilen medizinischer Instrumente**
Device for affixing medical instruments to cylindrical components
Dispositif destiné à la fixation à effet de blocage sur des composants cylindriques d'instruments médicaux

(30) Priorität: 23.11.2006 DE 102006055172
(43) Veröffentlichungstag der Anmeldung: 28.05.2008
(73) Patentinhaber: Karl Storz GmbH & Co. KG, 78532 Tuttlingen (DE)
(72) Erfinder: Frey, Sebastian, 78054 Villingen-Schwenningen (DE); Sauer, Michael, 78532 Tuttlingen (DE)
(74) Vertreter: Hofmeister, Frank

(56) Entgegenhaltungen:
- EP-A- 1 832 220
- WO-A-2006/132955
- US-A- 5 456 673
- US-A- 5 716 369

## Beschreibung

Die Erfindung betrifft eine Vorrichtung zum klemmenden Festlegen an zylindrischen Bauteilen medizinischer Instrumente, beispielsweise einem Instrumentenschaft, mit mindestens einer auf das zylindrische Bauteil aufsetzbaren und am zylindrischen Bauteil festlegbaren Aufnahme, wobei in einem gehäuse der Aufnahme eine das zylindrische Bauteil im Wesentlichen koaxial umgebende hülsenförmige Spannzange angeordnet ist, deren innerer Durchmesser zwischen einer entlang dem zylindrischen Bauteil verschiebbaren Weite und einer im Wesentlichen formschlüssig am zylindrischen Bauteil anliegenden Weite verstellbar ist.

Derartige Klemmvorrichtungen werden verwendet, um andere Bauteile, wie beispielsweise Handgriffe, Führungen oder auch medizinische Instrumente, an einem medizinischen Instrument festzulegen. Die Ausbildung der Fixierungsvorrichtung als Klemmvorrichtung hat sich in der Praxis bewährt, da diese in der Regel einfach zu handhaben und reversibel einsetzbar ist. Nachteilig bei den aus der Praxis bekannten Klemmvorrichtungen ist, dass aufgrund der Ausgestaltung des Klemmmechanismus die Gefahr besteht, dass das zylindrische Bauteil, an dem die Klemmvorrichtung angebracht werden soll, beschädigt wird. Diese Gefahr besteht insbesondere, wenn es sich bei dem zylindrischen Bauteil um ein optisches System, oder das Schaftrohr der Optik handelt.

Eine gattungsgemäße Klemmvorrichtung ist beispielsweise aus der DE 199 16 088 A1 bekannt. Bei dieser bekannten Klemmanordnung erfolgt das Festlegen des zu fixierenden medizinischen Instruments über zwei den zylindrischen Schaft des Instruments umgreifende Halbschalen, die über einen Exzenter so zusammendrückbar sind, dass die Halbschalen klemmend am Instrumentenschaft anliegen. Dieser Klemmmechanismus hat sich in der Praxis zwar durchaus bewährt, jedoch kann die Betätigung des Exzenterhebels insbesondere bei großen Schaftdurchmessern recht kraftaufwändig sein

Davon ausgehend liegt der Erfindung die Aufgabe zugrunde, eine Klemmvorrichtung der eingangs genannten Art zu schaffen, die bei einfacher Handhabung einerseits eine lagesichere Klemmung ermöglicht und andererseits eine schonende Klemmung des zylindrischen Bauteils gewährleistet.

Die Lösung dieser Aufgabenstellung ist erfindungsgemäß **dadurch gekennzeichnet, dass** die Spannzange in einem Schieber gelagert ist und der innere Durchmesser der Spannzange über den im Wesentlichen quer zum zylindrischen Bauteil verschiebbaren im Gehäuse der Aufnahme gelagerten Schieber verstellbar ist.

Die Verwendung dieses Schiebers zum reversiblen Überführen der Spannzange von der offenen in die Klemmstellung stellt eine besonders einfach und schnell zu handhabende Verstellmöglichkeit dar, um ein Bauteil lage- und verdrehsicher an dem zylindrischen Bauteil zu fixieren.

Durch Ausbildung des Klemmelements der Aufnahme als das zylindrische Bauteil im Wesentlichen koaxial umgebende hülsenförmige Spannzange besteht die Möglichkeit, die auf das zylindrische Bauteil ausgeübte Klemmkraft durch Ausbildung einer sich axial erstreckenden Flächenpressung über den Umfang des zylindrischen Bauteils so zu verteilen, dass keine eindrückende oder einschnürende lokale Klemmung auftritt.

Um die Montage der erfindungsgemäßen Klemmvorrichtung zu erleichtern und sicherzustellen, dass der Schieber immer in der richtigen Position in das Gehäuse eingesetzt wird, sind erfindungsgemäß am Schieber und am Gehäuse der Aufnahme miteinander korrespondierende Führungs- und Positionierungselemente vorgesehen.

Gemäß einer bevorzugten Ausführungsform der Erfindung wird vorgeschlagen, dass zum reversiblen Überführen der Spannzange von der offenen in die Klemmstellung die Spannzange und der Schieber über eine Zapfen-Schlitz-Steuerung miteinander in Wirkverbindung stehen, wobei die Zapfen-Schlitz-Steuerung vorzugsweise aus mindestens einem an der Spannzange gelagerten Steuerzapfen besteht, der in eine im Schieber ausgebildete Führungsbahn eingreift. Zapfen-Schlitz-Steuerungen zeichnen sich dadurch aus, dass bei einfacher Fertigung eine zuverlässige Schaltung der über sie angesteuerten Bauteile gewährleisten.

Erfindungsgemäß ist die Führungsbahn des Schiebers so ausgebildet, dass in Axialrichtung des zylindrischen Bauteils betrachtet in der im Schieber ausgebildeten Führungsbahn ein Höhenversatz ausgebildet ist.

Weiterhin wird mit der Erfindung vorgeschlagen, dass die Spannzange axialverschiebbar im Gehäuse der Aufnahme gelagert ist, um die Spannzange zwischen ihrem beiden Endstellungen, nämlich der das zylindrische Bauteil freigebenden Offenstellung und der das zylindrische Bauteil klemmend ergreifenden Klemmstellung zu verstellen.

Gemäß einer bevorzugten Ausführungsform der Erfindung wird vorgeschlagen, dass die Klemmwirkung der Spannzange dadurch erzeugt wird, dass an der Spannzange ein Konus ausgebildet ist, der mit einem im Gehäuse der Aufnahme ausgebildeten Gegenkonus in Wirkverbindung bringbar ist, wobei die Spannzange vorzugsweise über den mindestens einen in der Führungsbahn des Schiebers gelagerten Steuerzapfen so axialverschiebbar ist, dass der Konus der Spannzange zwischen einer gegen den Gegenkonus des Gehäuses anlaufenden Stellung und einer den Gegenkonus des Gehäuse freigebenden Stellung verlagerbar ist.

Die Ausbildung der die eigentliche Klemmwirkung erzeugenden Bauteile als Konus und Gegenkonus stellt eine Ausgestaltungsform dar, einfach zu fertigen und zu handhaben ist. Über die Zapfen-Schlitz-Steuerung wird die den Konus aufweisende Spannzange beim Schließen der Klemmverbindung gegen den Gegenkonus gedrückt und beim Öffnen der Klemmverbindung fort von dem Gegenkonus gezogen.

Mit einer praktischen Ausführungsform der Erfindung wird weiterhin vorgeschlagen, dass der Gegenkonus an der Innenseite einer auf das Gehäuse der Aufnahme aufsetzbaren Abdeckkappe ausgebildet ist.

Um sicherzustellen, dass der Schieber bei abgenommener Abdeckkappe nicht aus dem Gehäuse der Aufnahme fallen kann, ist erfindungsgemäß im Gehäuse der Aufnahme ein Sicherungselement, insbesondere ein Sprengring, zur seitlichen Lagesicherung des Schiebers angeordnet.

Schließlich wird mit der Erfindung vorgeschlagen, dass im Gehäuse der Aufnahme mindestens ein Dichtungselement angeordnet ist, um den Spülkreislauf des medizinischen Instruments bei aufgeklemmtem Instrument abzudichten.

Weitere Merkmale und Vorteile der Erfindung ergeben sich anhand der zugehörigen Zeichnung, in der ein Ausführungsbeispiel einer erfindungsgemäßen Vorrichtung zum klemmenden Festlegen an zylindrischen Bauteilen medizinischer Instrumente nur beispielhaft dargestellt ist, ohne die Erfindung auf dieses Ausführungsbeispiel zu beschränken. In der Zeichnung zeigt:
- Fig. 1: eine perspektivische Seitenansicht einer an einem Instrumentenschaft festgelegten erfindungsgemäßen Vorrichtung zum klemmenden Festle- gen an zylindrischen Bauteilen medizinischer Instrumente;
- Fig. 2: eine Seitenansicht der Klemmvorrichtung gemäß Fig. 1, die geöffnete Stellung darstellend;
- Fig. 3: einen Schnitt entlang der Linie III-III gemäß Fig. 2;
- Fig. 4: eine Seitenansicht der Klemmvorrichtung gemäß Fig. 2, jedoch die Klemmstellung darstellend;
- Fig. 5: einen Schnitt entlang der Linie V-V gemäß Fig. 4;
- Fig. 6: eine perspektivische Vorderansicht der Darstellung gemäß Fig. 1, je- doch ohne vordere Abdeckkappe und
- Fig. 7: eine perspektivische Ansicht des Klemmteils der erfindungsgemäßen Klemmvorrichtung.

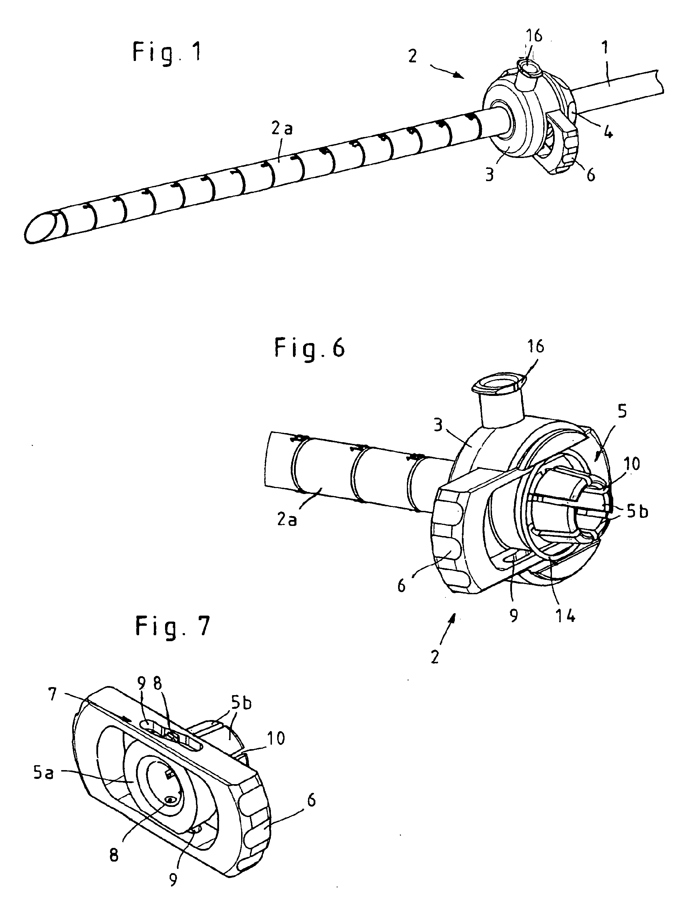

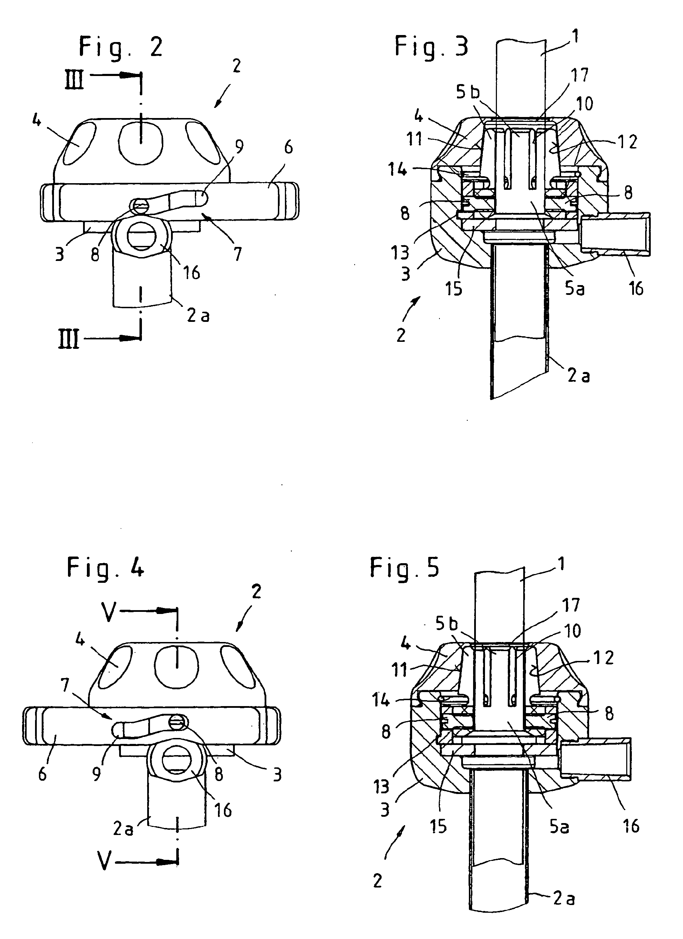

Die in den Abbildungen Fig. 1 bis 7 dargestellte Vorrichtung zum klemmenden Festlegen an zylindrischen Bauteilen 1 medizinischer Instrumente, wie beispielsweise Instrumentenschäften oder optischen Systemen endoskopischer Instrumente, besteht im Wesentlichen aus einer auf das zylindrische Bauteil 1 aufsetzbaren Aufnahme 2, in der der eigentliche Klemmmechanismus angeordnet ist. Die Aufnahme 2 besteht, wie insbesondere aus Fig. 3 und 5 ersichtlich, aus einem Gehäuse 3, das über eine Abdeckkappe 4 verschließbar ist.

Derartige Klemmvorrichtungen werden verwendet, um andere Bauteile, wie beispielsweise Handgriffe, Führungen oder auch medizinische Instrumente, an einem medizinischen Instrument festzulegen.

Bei der dargestellten Ausführungsform weist die Aufnahme 2 ein angesetztes Außenrohr 2a zur Aufnahme des zu klemmenden zylindrischen Bauteils 1 auf. Es ist jedoch selbstverständlich auch möglich, die Aufnahme ohne dieses Außenrohr 2a auszubilden, so dass das klemmend zu ergreifende zylindrische Bauteil 1 sich in Längsrichtung durch die Aufnahme 2 erstreckt..

Wie insbesondere aus den Abbildungen Fig. 3, 5 und 6 ersichtlich, besteht der Klemmmechanismus der Aufnahme 2 aus einer axialverschiebbar im Gehäuse 3 der Aufnahme angeordneten, hülsenförmigen Spannzange 5, die das zu klemmende zylindrische Bauteil 1 im Wesentlichen koaxial umgibt, sowie einem quer zum zylindrischen Bauteil 1 verschiebbar im Gehäuse 3 der Aufnahme 2 gelagerten Schieber 6, wobei die Spannzange 5 und der Schieber 6 über eine Zapfen-Schlitz-Steuerung 7 miteinander gekoppelt sind. Diese Zapfen-Schlitz-Steuerung 7 besteht bei der dargestellten Ausführungsform aus zwei an der Spannzange 5 gelagerten Steuerzapfen 8, die jeweils in einer im Schieber 6 ausgebildeten Führungsbahnen 9 gelagert sind, wobei die Führungsbahnen 9 in Axialrichtung des zylindrischen Bauteils 1 betrachtet ein Höhenversatz aufweisen.

Die Spannzange 5 ihrerseits besteht aus einem flachovalen Grundkörper 5a, von dem aus sich in axialer Richtung mehrere über Spalten 10 voneinander beabstandete Finger 5b koaxial um das zu klemmende zylindrische Bauteil 1 erstrecken. Die voneinander beabstandeten Finger 5b bilden dabei ein radial verformbares Federelement, über das eine Flächenpressung auf das zu klemmende zylindrische Bauteil 1 ausübbar ist. Die flachovale, das heißt unrunde Ausführung des Grundkörpers 5a dient zur Führung der Spannzange 5 im Schieber 6, wobei die Spannzange 5 über die abgeflachten Seiten des flachovalen Grundkörpers 5a verdrehsicher so im Schieber 6 gelagert ist, dass die Steuerzapfen 8 nicht aus den zugehörigen Führungsbahnen 9 herausrutschen können.

Der genaue Aufbau des Schiebers 6 sowie der Spannzange 5 ist insbesondere auch den Darstellungen gemäß den Abbildungen Fig. 6 und 7 zu entnehmen.

Zur Ausbildung der Klemmung über die Spannzange 5 ist an der Spannzange 5 ein Konus 11 ausgebildet, der mit einem im Gehäuse 3 der Aufnahme 2 ausgebildeten Gegenkonus 12 in Wirkverbindung bringbar ist. Bei der dargestellten Ausführungsform der Klemmvorrichtung bilden die Außenseiten der Finger 5b der Spannzange 5 den Konus 11, während an der Innenfläche der Abdeckkappe 4 der entsprechende Gegenkonus 12 ausgebildet ist.

Um einerseits die Montage der Aufnahme 1 zu erleichtern und anderseits das richtige Einsetzen des Schiebers 6 in das Gehäuse 3 zu gewährleisten, sind am Schieber 6 und am Gehäuse 3 der Aufnahme 2 miteinander korrespondierende Führungs- und Positionierungselemente 13 vorgesehen, die bei der in Fig. 3 und 5 dargestellten Ausführungsform als Nut-Feder-Verbindung ausgebildet sind. Wie weiterhin aus Fig. 3, 5 und 6 ersichtlich, ist im Gehäuse 3 ein als Sprengring ausgebildetes Sicherungselement 14 angeordnet, das bei abgenommener Abdeckkappe 4 ein Herausfallen des Schiebers 6 aus dem Gehäuse 3 verhindern soll.

Um den Spülkreislauf des medizinischen Instruments bei aufgeklemmtem Instrument abzudichten sind im Gehäuse 3 Dichtungselemente 15 angeordnet, die bei der dargestellten Ausführungsform unterhalb des Schiebers 6 im Gehäuse 3 angeordnet sind.

Die Handhabung der wie voranstehend beschrieben aufgebauten Vorrichtung zum klemmenden Festlegen an zylindrischen Bauteilen 1 medizinischer Instrumente arbeitet wie folgt:
Zu Beginn der Nutzung wird das zylindrische Bauteil 1 mit einem freien Ende voran in die Aufnahme 2 und das Außenrohr 2a der Klemmvorrichtung eingeschoben und nimmt dann die in Fig. 1 dargestellte Position ein. Bei der Darstellung gemäß Fig. 1 handelt es sich bei dem auf dem als Instrumentenschaft ausgebildeten zylindrischen Bauteil 1 festlegbaren Bauteil um ein mit einem Spülanschluss 16 versehenes Kopplungselement, über das mindestens ein weiteres Instrument mit dem Instrumentenschaft koppelbar ist.

Vor dem Aufschieben der Aufnahme 2 auf das zylindrische Bauteil 1 sowie unmittelbar danach befindet sich die Klemmvorrichtung in der in Fig. 2 und 3 dargestellten geöffneten Stellung, in der die Aufnahme in Axialrichtung frei verschiebbar sowie um die Längsachse verdrehbar auf dem zylindrischen Bauteil 1 angeordnet ist. In dieser geöffneten Stellung befinden sich die Steuerzapfen 8 der Zapfen-Schlitz-Steuerung 7 in der in Fig. 2 dargestellten unteren Position in der jeweiligen Führungsbahn 9.

Wie aus der zugehörigen Schnittdarstellung gemäß Fig. 3 ersichtlich, ist in dieser geöffneten Stellung der Spannzange 5 oberhalb der Finger 5b der Spannzange 5 ein Freiraum 17 zwischen der Oberkante der Abdeckkappe 4 und den freien Enden der Finger 5b ausgebildet. Die Ausbildung dieses Freiraums 17 bedeutet, dass der Konus 11 der Spannzange 5 noch nicht vollständig und folglich noch nicht klemmend in den Gegenkonus 12 der Abdeckkappe 4 eingeschoben wurde.

Zum klemmenden Festlegen der Aufnahme 2 am zylindrischen Bauteil 1 wird im nachfolgenden Arbeitsschritt der Schieber 6 ausgehend von der in Fig. 2 dargestellten rechten geöffneten Lage in die in Fig. 4 dargestellte linke Lage verschoben, die die Klemmstellung darstellt.

Aufgrund der Kopplung des Schiebers 6 und der Spannzange 5 über die Zapfen-Schlitz-Steuerung 7 bewirkt dieses Verschieben des Schiebers 6 im wesentlichen quer zur axialen Ausrichtung des zylindrischen Bauteils 1 ein Anheben der Steuerzapfen 8 in die in Fig. 4 dargestellte obere Position der Führungsbahn 9. Da die Steuerzapfen 8 fest mit der Spannzange 5 gekoppelt sind, bewirkt dieses Anheben der Steuerzapfen 8 zwangsläufig auch eine Axialverschiebung der Spannzange 5 in Richtung der Abdeckkappe 4.

Wie aus der zugehörigen Schnittdarstellung gemäß Fig. 5 ersichtlich, ist in dieser Klemmstellung der Spannzange 5 oberhalb der Finger 5b der Spannzange 5 so gut wie kein Freiraum 17 mehr zwischen der Oberkante der Abdeckkappe 4 und den freien Enden der Finger 5b ausgebildet. Dies bedeutet, dass der Konus 11 der Spannzange 5 nunmehr vollständig und folglich klemmend in den Gegenkonus 12 der Abdeckkappe 4 eingeschoben wurde. Aufgrund der über den Gegenkonus 12 auf die Finger 5b der Spannzange 5 ausgeübte seitliche Druckkraft werden die federelastischen Finger 5b im wesentlichen radial nach innen gedrückt, wodurch die Spannzange 5 das zylindrische Bauteil 1 nunmehr klemmend umgreift und die Klemmvorrichtung lagefest und verdrehsicher auf dem zylindrischen Bauteil fixiert.

Das Lösen der Klemmvorrichtung erfolgt in umgekehrter Reihenfolge durch Verschieben des Schiebers 6 aus der in Fig. 5 dargestellten linken Klemmstellung in die in Fig. 3 dargestellte rechte geöffnete Stellung.

Diese Verschiebung des Schiebers 6 bewirkt nunmehr ein Absenken der Steuerzapfen 8 der Zapfen-Schlitz-Steuerung 7 zurück in die untere Position in der jeweiligen Führungsbahn 9. Das Absenken der Steuerzapfen 8 bewirkt seinerseits einen Axialverschiebung der Spannzange 5 fort von der Abdeckkappe 4 und somit eine Trennung der klemmenden Anlage des Konus 11 der Spannzange 5 am Gegenkonus 12 der Abdeckkappe 4.

Die Aufnahme 2 der Klemmvorrichtung ist nunmehr wieder frei beweglich auf dem zylindrischen Bauteil 1 angeordnet und über ein freies Ende vom zylindrischen Bauteil 1 abgezogen werden.

Eine solchermaßen ausgebildete Klemmvorrichtung zeichnet sich dadurch aus, dass sie an beliebiger Stelle lagefest und verdrehsicher am zylindrischen Bauteil 1 festlegbar ist und die auf das zylindrische Bauteil 1 ausgeübte Klemmkraft durch Ausbildung einer sich axial erstreckenden Flächenpressung über den Umfang des zylindrischen Bauteils 1 so verteilt wird, dass keine eindrückende oder einschnürende lokale Klemmung auftreten kann.

### Bezugszeichenliste

- 1: zylindrisches Bauteil
- 2: Aufnahme
- 2a: Außenrohr
- 3: Gehäuse
- 4: Abdeckkappe
- 5: Spannzange
- 5a: Grundkörper
- 5b: Finger
- 6: Schieber
- 7: Zapfen-Schlitz-Steuerung
- 8: Steuerzapfen
- 9: Führungsbahn
- 10: Spalt
- 11: Konus
- 12: Gegenkonus
- 13: Führungs- und Positionierungselement
- 14: Sicherungselement
- 15: Dichtungselement
- 16: Spülanschluss
- 17: Freiraum

## Patentansprüche

1. Vorrichtung zum klemmenden Festlegen an zylindrischen Bauteilen medizinischer Instrumente, beispielsweise einem Instrumentenschaft, mit mindestens einer auf das zylindrische Bauteil (1) aufsetzbaren und am zylindrischen Bauteil (1) festlegbaren Aufnahme (2), wobei in einem Gehäuse (3) der Aufnahme (2) eine das zylindrische Bauteil (1) koaxial umgebende hülsenförmige Spannzange (5) angeordnet ist, deren innerer Durchmesser zwischen einer entlang dem zylindrischen Bauteil (1) verschiebbaren Weite und einer formschlüssig am zylindrischen Bauteil (1) anliegenden Weite verstellbar ist,
**dadurch gekennzeichnet,**
**dass** die Spannzange (5) in einem Schieber (6) gelagert ist und der innere Durchmesser der Spannzange (5) über den quer zum zylindrischen Bauteil (1) verschiebbar im Gehäuse (3) der Aufnahme (2) gelagerten Schieber (6) verstellbar ist.

2. Vorrichtung nach Anspruch 1, **dadurch gekennzeichnet, dass** am Schieber (6) und am Gehäuse (3) der Aufnahme (2) miteinander korrespondierende Führungs- und Positionierungselemente (13) vorgesehen sind.

3. Vorrichtung nach Anspruch 1 oder 2, **dadurch gekennzeichnet, dass** die Spannzange (5) und der Schieber (6) über eine Zapfen-Schlitz-Steuerung (7) miteinander in Wirkverbindung stehen.

4. Vorrichtung nach Anspruch 3, **dadurch gekennzeichnet, dass** die Zapfen-Schlitz-Steuerung (7) aus mindestens einem an der Spannzange (5) gelagerten Steuerzapfen (8) besteht, der in eine im Schieber (6) ausgebildete Führungsbahn (9) eingreift.

5. Vorrichtung nach Anspruch 4, **dadurch gekennzeichnet, dass** in Axialrichtung des zylindrischen Bauteils (1) betrachtet in der im Schieber (6) ausgebildeten Führungsbahn (9) ein Höhenversatz ausgebildet ist.

6. Vorrichtung nach einem der Ansprüche 1 bis 5, **dadurch gekennzeichnet, dass** die Spannzange (5) axialverschiebbar im Gehäuse (3) der Aufnahme (2) gelagert ist.

7. Vorrichtung nach einem der Ansprüche 1 bis 6, **dadurch gekennzeichnet, dass** an der Spannzange (5) ein Konus (11) ausgebildet ist, der mit einem im Gehäuse (3) der Aufnahme (2) ausgebildeten Gegenkonus (12) in Wirkverbindung bringbar ist.

8. Vorrichtung nach Anspruch 7, **dadurch gekennzeichnet, dass** die Spannzange (5) über den mindestens einen in der Führungsbahn (9) des Schiebers (6) gelagerten Steuerzapfen (8) so axialverschiebbar ist, dass der Konus (11) der Spannzange (5) zwischen einer gegen den Gegenkonus (12) des Gehäuses (3) anlaufenden Stellung und einer den Gegenkonus (12) des Gehäuse (3) freigebenden Stellung verlagerbar ist.

9. Vorrichtung nach Anspruch 7 oder 8, **dadurch gekennzeichnet, dass** der Gegenkonus (12) an der Innenseite einer auf das Gehäuse (3) der Aufnahme (2) aufsetzbaren Abdeckkappe (4) ausgebildet ist.

10. Vorrichtung nach einem der Ansprüche 1 bis 9, **dadurch gekennzeichnet, dass** im Gehäuse (3) der Aufnahme (2) ein Sicherungselement (14), insbesondere ein Sprengring, zur axialen Sicherung des Schiebers (6) angeordnet ist.

11. Vorrichtung nach einem der Ansprüche 1 bis 10, **dadurch gekennzeichnet, dass** im Gehäuse (3) der Aufnahme (2) mindestens ein Dichtungselement (15) angeordnet ist.

## Claims

1. Device for tight fixation on cylindrical components of medical instruments, for example, on an instrument shank with at least one receptacle (2) that can be attached to the cylindrical component (1) and is fixable at the cylindrical component (1), whereby in a casing (3) of the receptacle (2) a sleeve-like collet (5) is mounted that coaxially surrounds the cylindrical component (1), the inner diameter of which can be adjusted between the movable width along cylindrical component (1) and a form-fit closely fitting width at cylindrical component (1),
**characterized by,**
that the collet (5) is mounted in a slider (6) and the inner diameter of the collet (5) is adjustable by the slider (6) mounted displaceable horizontal to the cylindrical component (1) in casing (3) of receptacle (2).

2. Device according to Claim 1, **characterized by**, that at slider (6) and at casing (3) of the receptacle (2), guide and positioning elements (13) are provided that correspond to each other.

3. Device according to claim 1 or 2, **characterized by**, that the collet (5) and the slider (6) are in operative connection with each other by means of a pin-slot-control (7).

4. Device according to Claim 3, **characterized by**, that the pin-slot-control (7) consists of at least one control pin (8) that is mounted on the collet (5), which engages with a guide track (9) that is formed in slider (6).

5. Device according to Claim 4, **characterized by**, that viewed in the axial direction of the cylindrical component (1), a vertical offset is designed in the guide track (9) that is designed in slider (6).

6. Device according to one of claims 1 to 5, **characterized by**, that the collet (5) is mounted axially displaceable in casing (3) of receptacle (2).

7. Device according to one of claims 1 bis 6, **characterized by**, that at collet (5) a cone (11) is formed, that can be brought into operative connection with a counter cone (12) that is designed in the casing (3) of receptacle (2).

8. Device according to claim 7, **characterized by**, that the collet (5) is axially displaceable by the at least one control pin (8) located in the guide track (9) of the slider (6) in such a way, that the cone (11) of the collet (5) can be displaced between a position approaching the counter cone (12) of the casing (3) and a position that releases the counter cone (12) of the casing (3).

9. Device according to one of claims7 or 8, **characterized by**, that the counter cone (12) is formed on the inner side of a covering cap (4) that can be placed onto the casing (3) of receptacle (2).

10. Device according to one of claims 1 bis 9, **characterized by**, that in the casing (3) of receptacle (2) a retaining element (14) is mounted, in particular a snap ring, for axial locking of the slider (6).

11. Device according to one of claims 1 to 10, **characterized by**, that at least one sealing element (15) is located in casing (3) of receptacle (2).

## Revendications

1. Dispositif destiné à être fixé par serrage sur des pièces cylindriques d'instruments médicaux, par exemple sur un corps allongé d'instrument, comprenant une cage de réception (2) pouvant être rapportée sur la pièce cylindrique (1) et pouvant être fixée sur cette pièce cylindrique (1), dispositif dans lequel dans un carter (3) de la cage de réception (2) est agencée une pince de serrage (5) en forme de douille, qui entoure coaxialement la pièce cylindrique (1), et dont le diamètre intérieur peut être réglé entre une dimension permettant son coulissement le long de la pièce cylindrique (1), et une dimension pour laquelle elle s'applique par complémentarité de formes contre la pièce cylindrique (1),
**caractérisé en ce que** la pince de serrage (5) est montée dans un coulisseau (6), et le diamètre intérieur de la pince de serrage (5) peut être réglé par l'intermédiaire du coulisseau (6) monté dans le carter (3) de la cage de réception (2) en pouvant y coulisser transversalement par rapport à la pièce cylindrique (1).

2. Dispositif selon la revendication 1, **caractérisé en ce que** sur le coulisseau (6) et sur le carter (3) de la cage de réception (2) sont prévus des éléments de guidage et de positionnement (13) mutuellement en correspondance.

3. Dispositif selon la revendication 1 ou la revendication 2, **caractérisé en ce que** la pince de serrage (5) et le coulisseau (6) sont en interaction réciproque par l'intermédiaire d'une commande (7) à fente et tenon.

4. Dispositif selon la revendication 3, **caractérisé en ce que** la commande (7) à fente et tenon est réalisée par au moins un tenon de commande (8) monté sur la pince de serrage (5), qui vient en prise dans une voie de guidage (9) formée dans le coulisseau (6).

5. Dispositif selon la revendication 4, **caractérisé en ce que** vu dans la direction axiale de la pièce cylindrique (1), un décalage de niveau ou de hauteur est réalisé dans la voie de guidage (9) formée dans le coulisseau (6).

6. Dispositif selon l'une des revendications 1 à 5, **caractérisé en ce que** la pince de serrage (5) est montée axialement coulissante dans le carter (3) de la cage de réception (2).

7. Dispositif selon l'une des revendications 1 à 6, **caractérisé en ce que** sur la pince de serrage (5) est formé un cône (11) qui peut être amené en interaction avec un cône conjugué (12) formé dans le carter (3) de la cage de réception (2).

8. Dispositif selon la revendication 7, **caractérisé en ce que** par l'intermédiaire dudit au moins un tenon de commande (8) logé dans la voie de guidage (9) du coulisseau (6), il est possible de faire coulisser axialement la pince de serrage (5) de façon telle que le cône (11) de la pince de serrage (5) soit déplacé entre une position dans laquelle il est en appui contre le cône conjugué (12) du carter (3), et une position dégagée du cône conjugué (12) du carter (3).

9. Dispositif selon la revendication 7 ou la revendication 8, **caractérisé en ce que** le cône conjugué (12) est réalisé sur le côté intérieur d'un chapeau de recouvrement (4) pouvant être rapporté sur le carter (3) de la cage de réception (2).

10. Dispositif selon l'une des revendications 1 à 9, **caractérisé en ce que** dans le carter (3) de la cage de réception (2) est agencé un élément d'arrêt (14), notamment un jonc, pour assurer l'arrêt axial du coulisseau (6).

11. Dispositif selon l'une des revendications 1 à 10, **caractérisé en ce que** dans le carter (3) de la cage de réception (2) est agencé au moins un élément d'étanchéité (15).
